# EUROPEAN PATENT APPLICATION

(11) **EP 4 374 980 A1**
(43) Date of publication of application: **29.05.2024**
(21) Application number: 23211265.6
(22) Date of filing: 21.11.2023
(51) Int. Cl.: B09B 3/40, A61L 2/00, A61L 11/00, B02C 19/00, B09B 3/50, B09B 101/65

(54) **MACHINE FOR THE TREATMENT AND THE STERILIZATION OF HEALTHCARE WASTE**

(30) Priority: 23.11.2022 IT 202200024147
(71) Applicant: Camec S.r.l., 35013 Cittadella PD (IT)
(72) Inventor: SCIBILIA, Luciano Giovanni, 37040 Roveredo di Guà VR (IT)
(74) Representative: Modiano, Micaela Nadia

(57) **Abstract**

A machine (1) for the treatment and the sterilization of healthcare waste, which comprises a loading unit (4), a grinding unit (5), a sterilization unit (6) and a dumping unit (7); the sterilization unit (6) comprises a confined environment (15), where healthcare waste to be treated is accommodated, dry heating means (23, 24), which are designed to subject the healthcare waste to a dry thermal sterilization treatment at a temperature comprised between 90 and 450°C, and moving means (16; 21), which are designed to move the healthcare waste during the sterilization treatment.

## Description

The present invention relates to a machine for the treatment and the sterilization of healthcare waste.

Healthcare waste is waste produced in structures that conduct hospital, medical and veterinary activities for prevention, diagnosis, treatment, rehabilitation and research, and are defined an "infection risk". Such waste comprises, for example, waste originating from infective isolation wards, single-use healthcare or veterinary materials contaminated by biological fluids, sharp and piercing waste items, etc.

Generally, to be correctly disposed of, healthcare waste is placed in special containers, usually supplied by professional disposal firms, by operators wearing protective gloves and, if necessary, protective bodysuits and masks.

Once placed in the special containers, suitably labeled, the healthcare waste is stored in storerooms located in the structures that produce the healthcare waste, and are subsequently transferred to disposal plants.

In particular, healthcare waste is subject to specific national and international regulations, which control how it is managed in terms of movement, conservation, packaging, transport, labeling, traceability and disposal.

In other words, the regulations prohibit mixing healthcare waste with other types of hazardous waste or with urban refuse or waste, or sending it to landfill.

The current system for managing healthcare waste is centered around disposal using incinerators, of which however there is an insufficient number in the country, and which furthermore are not even evenly distributed. Such a situation necessarily entails the circulation and transport of healthcare waste on relatively long routes.

From the above it is clear that how healthcare waste is currently managed is burdensome, anti-ecological and hazardous, and that the present system of transporting healthcare waste necessarily entails a considerable expenditure of resources, while simultaneously producing an additional environmental impact over and above the inherent impact of the healthcare waste.

The need was therefore felt to find a solution that makes it possible to manage healthcare waste on-site without, therefore, subjecting the healthcare waste to a system of transport and, thus, avoiding the drawbacks that transport entails. The need was also felt for such solution to be capable of converting the "status" of the healthcare waste from "infection risk" to "sterile" or "bacteriologically inert materials", without generating pollution and, at the same time, providing a circular system of production and reuse of such waste that is ecologically-friendly and sustainable.

The aim of the present invention is to provide a machine, the technical characteristics of which are capable of meeting the requirement described above.

The subject matter of the present invention is a machine for the treatment of waste that substantially comprises a loading unit, a grinding unit and a sterilization unit, the main technical characteristics of which are given in the independent claim and the preferred or auxiliary characteristics of which are given in the dependent claims.

An embodiment of the present invention will be described below by way of non-limiting example, with the aid of the accompanying drawings wherein:
- Figure 1 is a perspective view of a preferred embodiment of the machine according to the present invention;
- Figure 2 is a first cross-sectional view of the machine of Figure 1 with parts removed for clarity;
- Figure 3 is a second cross-sectional view of the machine of Figure 1 with parts removed for clarity; and
- Figures 4 and 5 are respectively a plan view and a side view of alternative means of sterilization to those illustrated in the machine of Figures 1-3.

The present invention will now be described in detail with reference to the accompanying figures in order to enable a person skilled in the art to provide it and use it.

Except where otherwise defined, all the technical and scientific terms used herein have the same meanings as those commonly used by persons with ordinary experience in the sector to which the present invention refers.

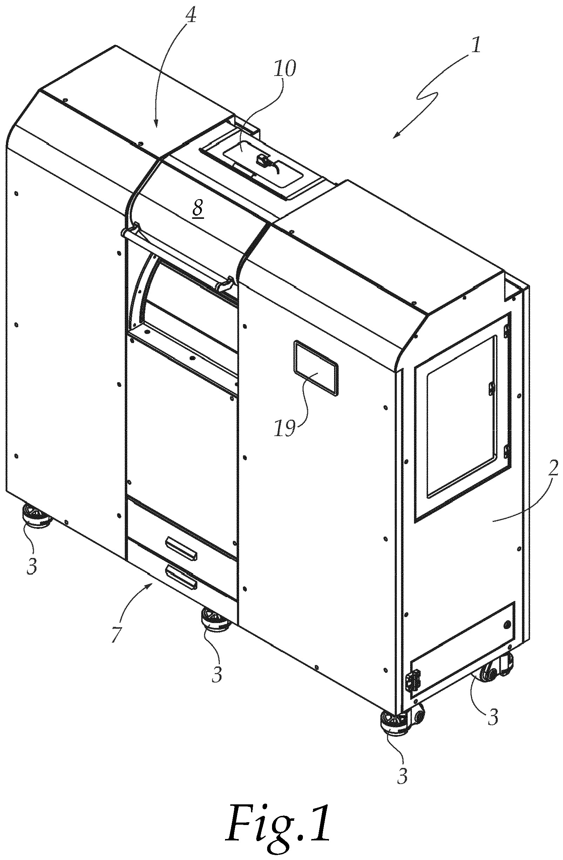

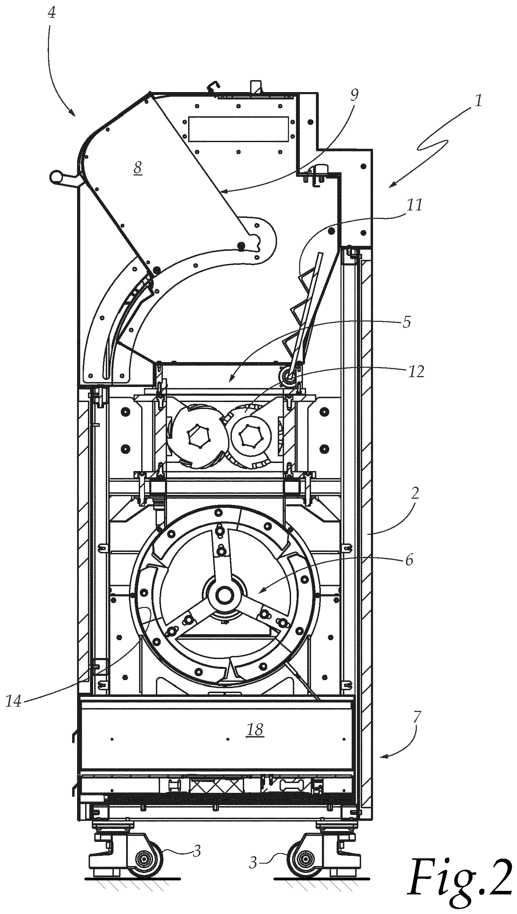

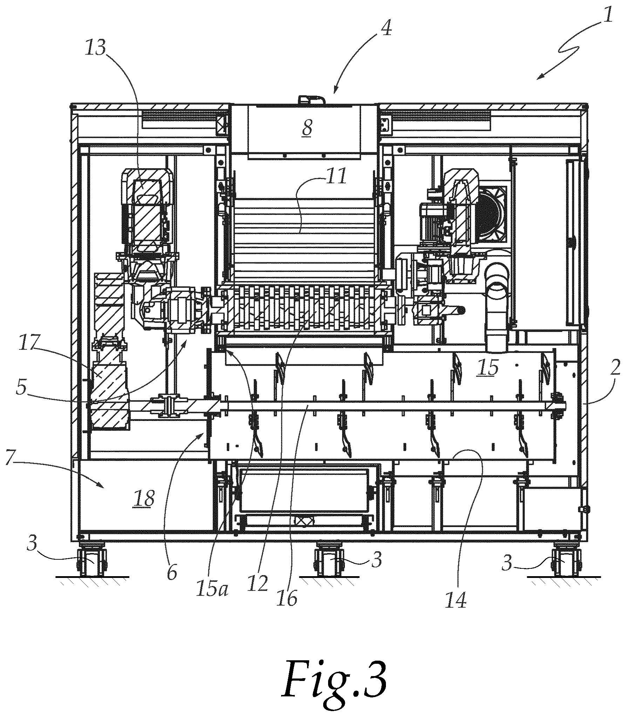

With reference to Figures 1-3, the reference numeral 1 generally designates a machine for the treatment and the sterilization of healthcare waste, the latter not being illustrated in that it is known.

The machine 1 comprises an electric power supply unit (not shown), for example photovoltaic panels, a containment structure 2 provided with wheels 3 in order to allow movement, a loading unit 4, a grinding unit 5, a sterilization unit 6 and a dumping unit 7. The grinding unit 5 and the sterilization unit 6 are fully accommodated inside the containment structure 2, while the loading unit 4 and the dumping unit 7 extend functionally outside the containment structure 2.

The outside walls of the containment structure 2 are treated with antibacterial paints, are insulated so as to not exceed 35°C, and are provided with acoustic insulation so as to limit the noise emissions of the machine 1 to below a relatively low threshold value.

The loading unit 4 comprises a loading hatch 8 which is arranged on the top of the machine 1 and can be moved by tilting. In an internal surface of the loading hatch 8 there is a loading tub 9, into which the bags containing the healthcare waste to be treated are placed.

In particular the operator, before placing the bags containing healthcare waste in the loading tub 9, scans the bags using a bar code reader, for the purposes of tracing the medical production area, the date the bag was sealed, the person responsible for the procedure, and other sensitive data defined by the producer of the waste.

Once the loading hatch 8 is brought to its closed position, the bags placed in the loading tub 9 are dropped into the grinding unit 5.

When the loading hatch 8 is in its "open" position the underlying area is kept in a constant partial vacuum by an aspiration system, which is conventional and not shown. Opening and closing the loading hatch 8 are commanded by the operator, with automatic activation of the aspirator. Furthermore, the confinement of the loading chamber has active locking systems that prevent it from being opened while the grinding unit is active, and there is a high-pressure UV bactericidal lamp which remains illuminated for the entire time of the cycle.

The loading unit 4 further comprises an inspection door 10, also provided in the top of the machine 1 and adapted to allow the operator to check for the presence of any waste that cannot be handled by the grinding unit 5 and which must, as a consequence, be removed.

The grinding unit 5 comprises a dual shaft grinder 12 actuated by an actuation device 13.

Each shaft of the grinder 12 is provided with a plurality of blades which cooperate with the blades of the other shaft in order to provide the grinding of the healthcare waste.

Alternatively, the grinder can be of the single shaft type aided by a hydraulically-actuated pusher that automatically feeds the material to be ground down.

The grinding unit 5 comprises a grille (not shown) positioned under the grinder 12 which has square mesh measuring from 10 to 100 mm on a side according to the dimensions of the fraction and the production requirements of the material being processed.

Furthermore, the grinding unit 5 comprises a glass-breaking confinement shutter 11 as an aid to the grinder 12. The glass-breaking confinement shutter 11 can move between a lifted open position in which it allows the passage of the healthcare waste to be treated, and a lowered closed position in which it pushes objects into the grinder 12 that owing to their shape or weight are not attacked by the plurality of blades. A further function of the glass-breaking shutter 11 in its closed position is to be adjacent to the grinder, so that it is subjected to sterilization by the sterilization unit 6.

The sterilization unit 6 is accommodated directly below the grinding unit 5 so as to receive the ground-up healthcare waste directly from it by gravity.

The sterilization unit 6 comprises a cylindrical wall 14 which defines a sterilization chamber 15 inside it and in which an inlet opening 15a is provided for receiving the healthcare waste directly from the grinding unit 5.

The sterilization unit 6 comprises a heating device (not shown), in this embodiment composed of electric resistance heaters, which is adapted to provide a temperature comprised between approximately 110°C and 400°C inside the sterilization chamber.

The sterilization unit 6 further comprises a vane mixer 16 mounted so as to rotate under the thrust of a gear motor 17. The vane mixer 16 is moved with an alternating rotary motion in order to shift the healthcare waste with a relative alternating straight motion.

Sterilization of the refuse or waste is a process that results in the abatement of the bacterial load (bioburden), and as a sterilizing agent it uses an effective combination of time and temperature in order to ensure a sterilization cycle that must be comparable, within established limits, to a previously validated cycle. The automatic sequence of operations inside the sterilization chamber 16 (heating, mixing and evaporation of the relative humidity of the waste) results in waste that is physically and biologically sterile. When the mixed waste has reached the sterilization parameters set in the control software, the concluded cycle is declared "valid" and then the waste is pushed toward the dumping unit 7. Differently, if the cycle does not confirm to the set sterilization levels, the cycle will be declared "aborted" and the sterilization procedure will therefore be repeated.

The sterilization chamber will be provided with connectors for collecting the sterilized material and performing tests. To verify the effectiveness and efficiency of the sterilization unit 6, a control procedure has been defined which involves contaminating a test load and analyzing it at the end of the sterilization operating cycle. For example, direct contamination of the test load is done using ampoules containing, for example, growth medium, sugar, a pH indicator, and spores of contaminant/bioindicator elements. The ampoules give coded results, for example with a color code, in particular a red/purple code to indicate a correct sterilization and a yellow/orange code to indicate an insufficient sterilization. The reliability of the response obtained has made it possible to monitor the sterilization process in a simple and rapid manner.

Once sterilized, the healthcare waste is pushed by the vanes of the mixer 16 to a dumper opening 15b located at the end of the sterilization chamber 16 and facing the dumping unit 7.

The dumping unit 7 comprises substantially a dumping drawer 18 which is adapted, when closed, to collect the sterilized healthcare waste and, when open, to allow the waste to be removed.

From the above it can be clearly seen that the sterilized healthcare waste is shifted so that it then falls due to gravity into the dumping drawer 18.

From the foregoing it can be seen that the loading unit 4, the grinding unit 5, the sterilization unit 6 and the dumping unit 7 are arranged with respect to each other along a substantially vertical direction, in such a way that the waste advances directly by gravity from the loading unit 4 to the dumping unit 7.

The machine 1 comprises an electronic control unit (not shown) which is configured to detect the step at any moment of the operating cycle (loading, grinding, sterilization, dumping), the duration of the step, the date and time the cycle ends, the completion and validity of the operating cycle. Furthermore, the electronic control unit is configured to record each operating cycle and to print, on request, a report on that operating cycle containing, in particular, data relating to the identification of the maker, the date and time, the serial number of the machine 1, the identification code of the selected sterilization program, the sequence number of the operating cycle, the weight of the healthcare waste, the place of production of the healthcare waste, and the presence of any radioactive healthcare waste. The electronic control unit is associated with a touchscreen monitor 19, through which the operator enters data, verifies the operation of the machine 1 and receives messages of completed sterilization, i.e. for a valid cycle or an aborted cycle. The touchscreen monitor 19 therefore makes it possible for the operator to select the sterilization cycle and to display the steps of the selected operating cycle.

The machine 1 is provided with both active and passive safety measures. In particular, the compartments for the transit and conversion of the healthcare waste are watertight.

The machine 1 further comprises a plurality of sensors (not shown) for monitoring the correct performance of the sterilization cycle, and a plurality of light emitting devices (not shown), analog and/or digital, which are adapted to indicate to the operator, for example, if the loading hatch 8 is closed, if the cycle is underway, if the cycle is completed, if the cycle is aborted, the number of operating cycles, and the status at any moment of the steps of the cycle.

The machine 1 further comprises:
- a reading device (not shown), which reads identification codes applied to the healthcare waste loaded in the loading tub 9 and registers information about the place of production of the healthcare waste;
- a drainage device (not shown) in the cylindrical wall 14 for draining a residue deriving from the sterilization of the healthcare waste;
- a weighing device (not shown) for weighing the healthcare waste; and/or
- a distribution device (not shown) for feeding deodorant agents into the healthcare waste sterilized in the sterilization unit 6.

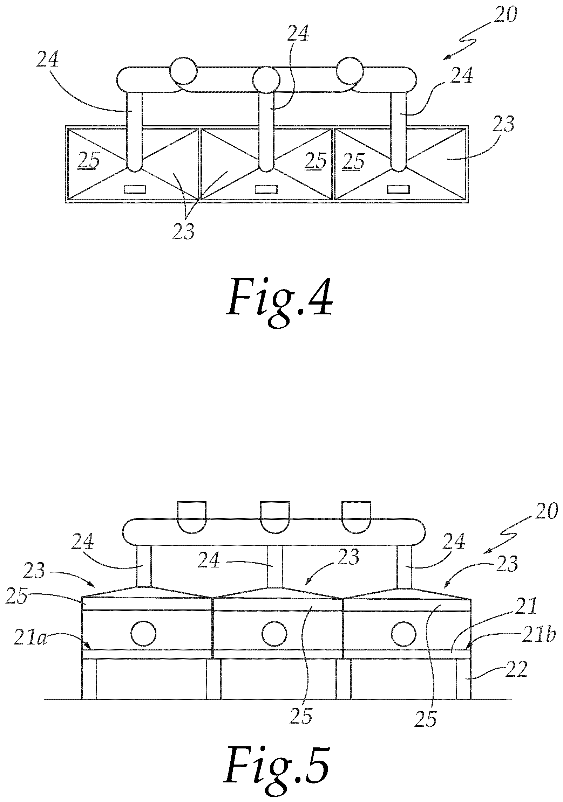

As an alternative to the means of sterilization described above, the machine according to the invention comprises the means of sterilization illustrated in Figures 4 and 5. Figures 4 and 5 show a more advantageous sterilization assembly of the machine according to the present invention.

In Figures 4 and 5 the number 20 designates a sliding sterilization assembly. The sliding sterilization assembly 20 comprises a conveyor belt 21 on which the healthcare waste deriving from the grinding unit 5 is deposited. The conveyor belt 21 is supported by a supporting structure 22 which is provided with special moving means (conventional and not shown). The sterilization assembly 20 further comprises three sources of infrared radiation 23 arranged in series above and along the conveyor belt 21, onto the upper surface of which they direct their heating action. In this manner, the waste is deposited on one end of the conveyor belt 21 and, during the movement of the belt, it is subjected sequentially to the action of the three sources of infrared radiation 23.

The three sources of infrared radiation 23 release heat at three different temperatures. In particular, the temperatures of the sources of infrared radiation 23 decrease in the direction of movement of the conveyor belt 21 and, therefore, of advancement of the waste.

In other words, the waste is deposited at an input end 21a of the conveyor belt 21, is subjected to heating by the sources of infrared radiation 23 by virtue of the movement of the conveyor belt which slides under the sources of infrared radiation 23 and, finally, is dropped from the output end 21b directly into the dumping unit 7.

Each one of the three sources of infrared radiation 23 is assisted by an air conveyance duct 24 and a collection hood 25 fixed at one end of the duct 24. The other end of the duct 24 is connected to an air movement system, not shown. The heating of the air is achieved by a plurality of electric resistance heaters which emit infrared radiation. The three collection hoods 25 are arranged in series, one after the other, and define three stations of decreasing temperature on the conveyor belt 21, which are passed through by the waste to be sterilized.

The three sources of infrared radiation 23 heat the air respectively to temperatures of 400°C, 160°C and 120°C. The sequential action of these three temperatures ensures the abatement of the bacterial load in the waste in a considerably shorter time than that required using the sterilization chamber 15 with the vane mixer 16. In fact, it has been calculated experimentally that with the sliding sterilizer 20 it is possible to sterilize 2500 Kg/h of waste, against the 150 Kg/h that can be sterilized with the sterilization chamber 15.

The peculiarity of the sliding sterilization assembly 20 is that it allows elevated heating and, as a consequence, it enables shorter treatment times. In this regard, with the sliding sterilization assembly 20 the sterilization cycle has a time of approximately 6 minutes.

The sterilization operating cycle executed by the machine 1 comprises the steps of:
- weighing the healthcare waste;
- depositing the healthcare waste, placed inside special packaging bags, into the loading tub 9;
- detecting the identification codes applied on the packaging bags;
- closing the loading hatch 9, activating the detection device (not shown) in order to detect the presence of any radioactive healthcare waste, and activating the UV lamp (not shown) to sanitize the loading unit 4 overall;
- verifying the presence of waste to be removed;
- feeding deodorant agents into the healthcare waste;
- grind up the healthcare waste with the grinding unit 5 when the absence of any healthcare objects to be removed has been ascertained;
- transferring the healthcare waste to the sterilization unit 6;
- activating the sterilization, setting the treatment temperatures; and
- sending the sterilized healthcare waste into the dumping unit 7 and, then, into the dumping drawer 18.

The advantage of the machine 1 is that it effectively treats healthcare waste directly on the site of its production and it is at the same time particularly simple to manage.

In this manner the machine 1 does not need the use of resources and manpower storing and transporting healthcare waste, as a consequence reducing the associated environmental impact, and also does not need highly specialist staff to execute the sterilization treatment.

Summing up, the machine according to the present invention makes it possible in an effective and simple manner to convert potentially infectious healthcare waste to bacteriologically inert materials directly at the site of their production.

The disclosures in Italian Patent Application No. 102022000024147 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A machine (1) for the treatment and the sterilization of healthcare waste, which comprises a loading unit (4), a grinding unit (5), a sterilization unit (6) and a dumping unit (7), **characterized in that** said sterilization unit (6) comprises a confined environment (15), where healthcare waste to be treated is accommodated, dry heating means (23, 24), which are designed to subject the healthcare waste to a dry thermal sterilization treatment at a temperature comprised between 90 and 450°C, and moving means (16; 21), which are designed to move the healthcare waste during the sterilization treatment.

2. The machine according to claim 1, **characterized in that** said heating means comprise a plurality of heat sources (23) with a temperature that decreases along an advancement direction of the healthcare waste.

3. The machine according to claim 2, **characterized in that** said heat sources (23) emit infrared radiation at a temperature comprised between 450°C and 90°C.

4. The machine according to claim 2, **characterized in that** said moving means comprise a conveyor belt (21), on which the healthcare waste is deposited, said heat sources (23) being arranged sequentially above a longitudinal extension of the conveyor belt (21) and being designed to produce a heat that decreases along the advancement direction of the healthcare waste.

5. The machine according to claim 2, **characterized in that** said heat sources comprise a first heat source (23) adapted to emit infrared radiation at a temperature comprised between 350°C and 450°C, a second heat source (23) adapted to emit infrared radiation at a temperature comprised between 100°C and 200°C, a third heat source (23) adapted to emit infrared radiation at a temperature comprised between 90°C and 160°C.

6. The machine according to claim 1, **characterized in that** said loading unit (4) is arranged above and is directly connected to said grinding unit (5), which is arranged above and is directly connected to said sterilization unit (6), which is arranged above and is directly connected to said dumping unit (7).

7. The machine according to claim 1, **characterized in that** said loading unit (4) comprises an inspection door (10) which is designed to allow operators to check for the presence of any waste to be removed.

8. The machine according to claim 1, **characterized in that** said grinding unit (5) also comprises a glass-breaking confinement shutter (11); said glass-breaking confinement shutter (11) being movable between a lifted open position, in which it allows the passage of the healthcare waste to be treated, and a lowered closed position, in which it pushes said healthcare waste into a grinder (12).
